# EUROPEAN PATENT APPLICATION

(11) **EP 2 159 259 A1**
(43) Date of publication of application: **03.03.2010**
(21) Application number: 09011055.2
(22) Date of filing: 28.08.2009
(51) Int. Cl.: C08L 5/08, C08L 89/00, C08L 89/06

(54) **A structure comprising chitosan and collagen**

(30) Priority: 28.08.2008 JP 2008220099
(71) Applicant: Fujifilm Corporation, Tokyo 106-0031 (JP)
(72) Inventor: Shimada, Toshio, Kaisei-machi, Ashigarakami-gun Kanagawa 258-8538 (JP)
(74) Representative: Siegert, Georg

(57) **Abstract**

It is an object of the present invention to provide a structure comprised of a mixture of chitosan and collagen, which achieves both degradability and tissue adhesiveness. The present invention provides a structure comprised of a mixture of chitosan and collagen, wherein (i) the deacetylation degree of chitosan is 50% to 70%, (ii) the weight ratio of chitosan and collagen is 30/70 or more and less than 70/30.

SEQUENCE LISTING

<110> FUJIFILM Corporation

<120> A structure comprising chitosan and collagen

<130> FA9133A

<150> JP2008-220099
<151> 2008-08-28

<160> 1

<210> 1
<211> 576
<212> PRT
<213> Artificial

<220>
<223> Recombinant gelatin

<400> 1
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
            20                  25                  30
Arg Gly Asp Ala Gly Pro Lys Gly Ala Asp Gly Ala Pro Gly Ala Pro
        35                  40                  45
Gly Leu Gln Gly Met Pro Gly Glu Arg Gly Ala Ala Gly Leu Pro Gly
    50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
        115                 120                 125
Arg Gly Leu Ala Gly Pro Ile Gly Pro Pro Gly Pro Ala Gly Ala Pro
    130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
            180                 185                 190
Gly Ala Pro Gly Ala Pro Gly Leu Gln Gly Ala Pro Gly Leu Gln Gly
        195                 200                 205
Met Pro Gly Glu Arg Gly Ala Ala Gly Leu Pro Gly Pro Lys Gly Glu
    210                 215                 220
Arg Gly Asp Ala Gly Pro Lys Gly Ala Asp Gly Ala Pro Gly Ala Pro
225                 230                 235                 240
Gly Leu Gln Gly Met Pro Gly Glu Arg Gly Ala Ala Gly Leu Pro Gly
                245                 250                 255
Pro Lys Gly Glu Arg Gly Asp Ala Gly Pro Lys Gly Ala Asp Gly Ala
            260                 265                 270
Pro Gly Lys Asp Gly Val Arg Gly Leu Ala Gly Pro Ile Gly Pro Pro
        275                 280                 285
Gly Glu Arg Gly Ala Ala Gly Leu Pro Gly Pro Lys Gly Glu Arg Gly
    290                 295                 300
Asp Ala Gly Pro Lys Gly Ala Asp Gly Ala Pro Gly Lys Asp Gly Val
305                 310                 315                 320
Arg Gly Leu Ala Gly Pro Ile Gly Pro Pro Gly Pro Ala Gly Ala Pro
                325                 330                 335
Gly Ala Pro Gly Leu Gln Gly Met Pro Gly Glu Arg Gly Ala Ala Gly

            340                 345                 350
Leu Pro Gly Pro Lys Gly Glu Arg Gly Asp Ala Gly Pro Lys Gly Ala
        355                 360                 365
Asp Gly Ala Pro Gly Lys Asp Gly Val Arg Gly Leu Ala Gly Pro Pro
    370                 375                 380
Gly Ala Pro Gly Ala Pro Gly Leu Gln Gly Ala Pro Gly Leu Gln Gly
385                 390                 395                 400
Met Pro Gly Glu Arg Gly Ala Ala Gly Leu Pro Gly Pro Lys Gly Glu
                405                 410                 415
Arg Gly Asp Ala Gly Pro Lys Gly Ala Asp Gly Ala Pro Gly Ala Pro
            420                 425                 430
Gly Leu Gln Gly Met Pro Gly Glu Arg Gly Ala Ala Gly Leu Pro Gly
        435                 440                 445
Pro Lys Gly Glu Arg Gly Asp Ala Gly Pro Lys Gly Ala Asp Gly Ala
    450                 455                 460
Pro Gly Lys Asp Gly Val Arg Gly Leu Ala Gly Pro Ile Gly Pro Pro
465                 470                 475                 480
Gly Glu Arg Gly Ala Ala Gly Leu Pro Gly Pro Lys Gly Glu Arg Gly
                485                 490                 495
Asp Ala Gly Pro Lys Gly Ala Asp Gly Ala Pro Gly Lys Asp Gly Val
            500                 505                 510
Arg Gly Leu Ala Gly Pro Ile Gly Pro Pro Gly Pro Ala Gly Ala Pro
        515                 520                 525
Gly Ala Pro Gly Leu Gln Gly Met Pro Gly Glu Arg Gly Ala Ala Gly
    530                 535                 540
Leu Pro Gly Pro Lys Gly Glu Arg Gly Asp Ala Gly Pro Lys Gly Ala
545                 550                 555                 560
Asp Gly Ala Pro Gly Lys Asp Gly Val Arg Gly Leu Ala Gly Pro Pro
                565                 570                 575

## Description

### Technical Field

The present invention relates to a structure comprising chitosan and collagen.

### Background Art

In the industrial field, chitosan is an aminopolysaccharide, which is mainly obtained by deacetylation of chitin obtained from the exoskeletons of crustaceans such as crab and shrimp by a boiling treatment or the like in concentrated alkali. In general, since chitosan is easily dissolved in an acetic acid aqueous solution and various types of molded products such as a film, a fiber and a sponge can be produced from the thus obtained solution, chitosan has been widely used in the fields of cosmetic products, medicines, and food products. It is preferably used as a natural material. Since chitosan has a cationic property, it has a high adhesion property to tissues. Thus, utilizing such property, chitosan has been used as a wound-coating agent or an adhesion preventive agent on the surface of a living body.

In particular, when chitosan is applied as a material used on the surface of a living body or in such living body, optimization of biological, chemical, physical, and structural factors is required. *In vivo* degradability obtained as a summation of such optimized factors is one of the most important factors for a material used *in vivo.* However, there may be some cases in which it is difficult for chitosan which is a polysaccharide not existing in a living body to keep such *in vivo* degradability within a preferred range, when it is used singly. Thus, the formation of a composite material consisting of chitosan and another polymer derived from an organism has been studied.

As an example, a composite material consisting of chitosan and collagen that is an organism-derived polymer has previously been studied. The use of such composite material of chitosan and collagen as an artificial skin, artificial blood vessel, would-coating material, or adhesion preventive agent has been studied (JP Patent Publication (Kokai) Nous. 56-133344 A (1981) and 63-59706 A (1988)). Generally, combination of chitosan with collagen improves *in vivo* degradability, but it decreases tissue adhesiveness. The achievement of both tissue adhesiveness and degradability is required for application of such composite material to biomaterials. The conventional chitosan/organsm-derived polymer composites have not achieved both tissue adhesiveness and degradability.

### Disclosure of the Invention

It is an object of the present invention to solve the aforementioned problem of the prior art technique. In other words, it is an object of the present invention to provide a structure comprised of a mixture of chitosan and collagen, which achieves both degradability and tissue adhesiveness.

As a result of intensive studies directed towards achieving the aforementioned object, the present inventors have found that a structure comprised of a mixture of chitosan and collagen, which achieves both degradability and tissue adhesiveness, can be produced by using chitosan having a deacetylation degree of 50% to 70%, and also setting the weight ratio of chitosan and collagen at 30/70 or more and less than 70/30, thereby completing the present invention.

Namely, the present invention provides a structure comprised of a mixture of chitosan and collagen, wherein (i) the deacetylation degree of chitosan is 50% to 70%, (ii) the weight ratio of chitosan and collagen is 30/70 or more and less than 70/30.

Preferably, the collagen is a recombinant collagen.

Preferably, the weight ratio of chitosan and collagen is 33/67 or more and 60/40 or less.

Preferably, the structure of the present invention is produced by dissolving chitosan having a deacetylation degree of 50% to 70% and a collagen in a solvent in such an amount that the weight ratio of chitosan and collagen is 30/70 or more and less than 70/30, so as to produce a solution, and drying the solution.

The present invention further provides a method for producing the structure of the present invention as mentioned above, which comprises dissolving chitosan having a deacetylation degree of 50% to 70% and a collagen in a solvent in such an amount that the weight ratio of chitosan and collagen is 30/70 or more and less than 70/30, so as to produce a solution, and drying the solution.

According to the present invention, it becomes possible to provide a structure comprised of a mixture of chitosan and collagen, which achieves degradability and tissue adhesiveness.

### Preferred Embodiment of the Invention

The present invention will be described more in detail below.

### <Chitosan>

Chitosan used in the present invention is a product obtained by deacetylation of aminopolysaccharide, chitin, contained in the exoskeletons of crustaceans such as crab and shrimp. As a chemical structure, chitosan is a natural polymer having glucosamine, or glucosamine and a small amount of N-acetylglucosamine, as a repeating unit. In general, chitosan is obtained by deproteinizing the exoskeletons of crustaceans with an alkaline aqueous solution, then eliminating calcium from the resultant with a hydrochloric acid aqueous solution to obtain chitin, and then deacetylating the obtained chitin with a high concentration alkaline aqueous solution such as caustic soda. In general, the deacetylation degree of chitosan can be arbitrarily adjusted. Such chitosan is not dissolved in water, but is dissolved in an aqueous solution containing an organic solvent such as acetic acid.

A common method for producing chitosan comprises: deproteinizing, for example, the shell of a fresh red snow crab with a dilute alkaline aqueous solution such as caustic soda; eliminating calcium from the resultant product with a dilute acid aqueous solution such as hydrochloric acid to obtain chitin; and treating the obtained chitin in a concentrated alkaline aqueous solution containing approximately 40 to 60 wt % alkaline for approximately 5 to 20 hours, while keeping a temperature of approximately 90°C or higher. The chitosan product obtained by the aforementioned method is generally in a solid flake state, and such chitosan product is generally dried and used in the form of powders. The obtained chitosan is a polymer, and even after it has been purified, it has a high molecular weight. Its molecular weight is generally between approximately 100,000 and 3,000,000. In general, such molecular weight can be measured by dissolving chitosan in a certain acetic acid aqueous solution and measuring the viscosity of the solution, and it can be then controlled. As a standard viscosity measurement, in general, 0.5 wt % chitosan is dissolved in an acetic acid aqueous solution, and the viscosity of the obtained solution is then measured at 20°C using a B-type viscometer. The viscosity of ordinary chitosan obtained by the aforementioned measurement method is generally 3 to 500 mPa·s. It is generally 10 to 250 mPa·s. For example, the solution viscosity of chitosan with a molecular weight of approximately 300,000 is approximately 10 mPa·s.

### <Collagen>

The term "collagen" is used in the present specification to mean collagen or gelatin, which has a GXY portion characteristic for collagen. The terms "collagen" may be sometimes mixed with the term "gelatin" in the present specification. However, the two terms have the same above definition. In comparison with other proteins, the GXY portion characteristic for collagen is an extremely specific partial structure existing in the amino acid composition and amino acid sequence of gelatin or collagen. In this portion, glycine accounts for approximately one-third of all amino acids. In the amino acid sequence, glycine appears repeatedly at a rate of one of three amino acids. Glycine is the simplest amino acid. It hardly constrains the conformation of molecular chain, and greatly contributes to the regeneration of a helix structure in gelation. In amino acids represented by X and Y, large quantities of imino acids (prolines and oxyprolines) are contained. Such imino acids account for 10% to 45% of all amino acids. The origin of the protein is not particularly limited. Any protein of human, bovine, swine or fish and gene recombinant protein may be used.

With regard to a gene recombinant collagen used in the present specification, methods and products described in, for example, EP0926543B, WO02/052342, EP1063565B, WO2004085473 EP1014176A, and U.S. Patent No. 6,992,172, may be used, depending on purposes.

The gene recombinant collagen used in the present invention is naturally excellent in terms of biocompatibility and noninfectiousness. In addition, the gene recombinant collagen used in the present invention is homogeneous, when compared with natural collagen. The sequence of the gene recombinant collagen has been determined. Thus, it becomes possible to precisely design the gene recombinant collagen by the below-mentioned crosslinking or the like with less deviation with respect to strength and degradability.

As a gene recombinant collagen used in the present specification, collagen having the following characteristic (1) and collagen having the following characteristic (2) are particularly preferable.
(1) Gene recombinant gelatin, in which the percentage of polar amino acids to all constituent amino acids is 30% to 60% and the percentage of noncharged amino acids to the polar amino acids is 70% or more.
   The present inventors have found that the biodegradability of a gelatin composition can be suppressed using a gene recombinant gelatin having the aforementioned amino acid sequence. In common gelatin, charged polar amino acids and noncharged polar amino acids are present at a ratio of 1 : 1. The term "polar amino acid" is used herein to specifically mean cysteine, aspartic acid, glutamic acid, histidine, lysine, asparagine, glutamine, serine, threonine, and tyrosine. Of these, the term "noncharged polar amino acid" is used to mean cysteine, asparagine, glutamine, serine, threonine, and tyrosine. Preferred polar amino acids are serine, glutamine, and asparagine. The percentage of such preferred polar amino acids is more preferably 30% to 60% with respect to all amino acids.
   Moreover, in gelatin, glutamine composition in noncharged polar amino acids is preferably 30% or more and 80% or less, and more preferably 40% or more and 60% or less. Furthermore, the percentage of hydroxyl groups to primary amides on side chains in noncharged polar amino acids is preferably 50% or less, and more preferably 30% or less. More specifically, the percentage of the sum of the number of serine and threonine residues to the number of glutamine residues is 60% or less, and more preferably 40% or more and 60% or less. The percentage of the number of serine residues to the number of glutamine residues is more preferably 40% or more and 60% or less, and further preferably 50%.
(2) Gelatin which is **characterized in that** its amino acid sequence comprises 3 to 50 RGD sequences in a single molecule.
   The present inventors have found that the biodegradability of a gelatin composition can be promoted by using a gene recombinant gelatin having the aforementioned amino acid sequence.
   In general, an RGD sequence has been known as a minimal amino acid sequence that acts as a cell adhesion signal in a polypeptide (see "Byotai Seiri (Pathologic Physiology)," Vol. 9, No. 7 (1990), p. 527, published by Nagai Shuppan K.K., for example). In the gene recombinant gelatin used in the present invention, preferably 3 to 50, more preferably 4 to 30, and particularly preferably 5 to 20 RGD sequences are contained in a single molecule. In the gene recombinant gelatin used in the present invention, the object has been achieved by controlling and expressing such sequence.

The gene recombinant gelatin used in the present invention, which includes the gene recombinant gelatins described in (1) or (2) above, are substantially pure collagen materials prepared by using a nucleic acid which encodes natural collagen. Such gene recombinant gelatin may be partially hydrolyzed. The gelatin may have amino acid identity of 40%, and more preferably 50% or more with the sequence of an organism-derived collagen. The aforementioned amino acid identity is further preferably 80% or more, and most preferably 90% or more. As collagen used herein, any type of collagen may be used, as long as it is present in nature. Generally, the type of a necessary sequence largely differs depending on therapeutic purpose. That is, a sequence close to the sequence of collagen necessary for treating relevant tissues is desirable. For example, in the case of a material surface for treating the cartilage, the sequence of type II collagen is desirable. In the case of blood vessel, type I collagen is preferable for the adventitia, and type IV collagen is preferable for the intima. Type I, type II, type III, type IV, and type V collagens are preferable. Type I, type II, and type III collagens are more preferable. In another embodiment, such collagen is preferably derived from a human, a bovine, a swine, a mouse, or a rat. The origin of such collagen is more preferably a human. Most preferably, such collagen has homology of 80% or more with the amino acid sequence of human collagen α1. In addition, the isoelectric point of the gene recombinant gelatin is 4 to 10, preferably 6 to 10, and more preferably 7 to 9. The gene recombinant gelatin has a GXY portion characteristic for collagen, and it has a molecular weight of 2 KDa or more and 100 KDa or less, more preferably 2.5 KDa or more and 95 KDa or less, further preferably 5 KDa or more and 90 KDa or less, and most preferably 10 KDa or more and 90 KDa or less.

When a single use of such gene recombinant gelatin exhibits insufficient performance, the gene recombinant gelatin may be mixed or combined with other materials. For example, the gene recombinant gelatin may be mixed with natural gelatin, a different type of gene recombinant gelatin, another biopolymer, or a synthetic polymer. Examples of such biopolymer include a polysaccharide, a polypeptide, a protein, a nucleic acid, and an antibody. A polysaccharide, a polypeptide, and a protein are preferable. Examples of such polysaccharide, polypeptide, and protein include glucosaminoglycan such as hyaluronic acid and heparin. chitin, chitosan, poly-γ-glutamic acid, collagen, gelatin, albumin, fibroin, and casein. These compounds may be partially chemically modified, as necessary. For example, hyaluronic acid ethyl ester may be used.

The gene recombinant gelatin used in the present invention may be chemically modified, depending on intended use. Such chemical modification includes: introduction of a low molecular weight compound or various types of polymers (a biopolymer (sugar or a protein), a synthetic polymer, or a polyamide) into a carboxyl group or an amino group on the side chain of the gene recombinant gelatin; and crosslinking between the gene recombinant gelatins. An example of such introduction of a low molecular weight compound into the gene recombinant gelatin is the use of a carbodiimide condensation agent.

In the present invention, gelatin may be crosslinked. Such crosslinking may be preferably carried out using heat, light, a condensation agent, or enzyme. The crosslinking agent used in the present invention is not particularly limited, as long as the present invention can be carried out in the presence of such crosslinking agent. Thus, either a chemical crosslinking agent or enzyme may be used. Examples of such chemical crosslinking agent include formaldehyde, glutaraldehyde, carbodiimide, and cyanamide. Of these, formaldehyde and glutaraldehyde are preferable, and glutaraldehyde is most preferable.

When crosslinking is carried out using enzyme, the type of such enzyme is not particularly limited, as long as it has action to crosslink between gene recombinant gelatin chains. Such crosslinking may be earned out, preferably using transglutaminase or laccase, and most preferably using transglutaminase. The specific type of a protein that is crosslinked with transglutaminase is not particularly limited, as long as it has a lysine residue and a glutamine residue. Such transglutaminase may be derived from either mammals or microorganisms. Specific examples of such transglutaminase include: Activa series manufactured by Ajinomoto Co., Inc.; transglutaminases derived from mammals that are available as reagents, such as transglutaminase derived from guinea pig liver, transglutaminase derived from goat, and transglutaminase derived from rabbit, which are manufactured by Oriental Yeast Co., Ltd., Upstate USA Inc., Biodesign International, etc.; and a human-derived blood coagulation factor (Factor XIIIa; Haematologic Technologies, Inc.). When the aforementioned gene recombinant gelatin is crosslinked with transglutaminase, crosslinking efficiency can be improved if a glutamine composition is high. Accordingly, the quantities of primary amide groups on the side chains of an amino acid are preferably 2 times or more, and more preferably 3 times or more the quantities of carboxylic acid groups. More specifically, with regard to primary amide groups, it is desired that the quantities of glutamine groups be higher than the quantities of glutamic acid groups. The quantities of glutamine groups are preferably 2 times or more, and more preferably 3 times or more the quantities of glutamic acid groups.

The process of crosslinking the gene recombinant gelatin includes two steps, namely, a step of mixing a gelatin solution with a crosslinking agent and a step of reacting a homogeneous solution of them.

The temperature for mixing gelatin with a crosslinking agent in the present invention is not particularly limited, as long as the solution can be homogeneously stirred at the temperature. Such mixing temperature is preferably 0°C to 40°C, more preferably 0°C to 30°C, further preferably 3°C to 25°C, further more preferably 3°C to 15°C, still further preferably 3°C to 10°C, and particularly preferably 3°C to 7°C.

After the gelatin and the crosslinking agent have been stirred, the temperature may be increased. The reaction temperature is not particularly limited, as long as the reaction progresses at the temperature. If taking into consideration the degeneration or decomposition of a biopolymer, the reaction temperature is substantially 0°C to 60°C, preferably 0°C to 40°C, more preferably 3°C to 25°C, further preferably 3°C to 15°C, further more preferably 3°C to 10°C, and particularly preferably 3°C to 7°C.

### <Composite material>

Next, the structure comprised of a mixture of chitosan and collagen of the present invention will be described. In the present invention, the present inventors have discovered an unexpected property of a composite of gelatin and chitosan having a deacetylation degree of 50% to 70%, preferably 55% to 65%, and more preferably 60% to 65%. Specifically, it is a phenomenon whereby tissue adhesiveness specifically increases within a range in which the weight ratio of chitosan and gelatin is 30/70 or more and less than 70/30, preferably 35/65 or more and 65/35 or less, more preferably 33/67 or more and 60/40 or less, further preferably 40/60 or more and 60/40 or less, and most preferably 45/55 or more and 55/45 or less. It had been assumed that, as the ratio of gelatin increases, tissue adhesiveness generally decreases. However, it was totally unexpected that tissue adhesiveness can be improved at the weight ratio of chitosan/gelatin = approximately 50/50, when the deacetylation degree of the chitosan is set at 50% to 70%. The same tissue adhesiveness can be also achieved in a case in which the ratio of chitosan is high. In such a case, however, the ratio of gelatin is low, and thus it is problematic in terms of degradability.

Adhesion between the aforementioned composite structure and tissues can be quantified by employing a commonly used tensile test machine. In order to prevent the peeling of the composite structure after it has been attached, adhesion energy is preferably 1.5 mJ or more, and more preferably 2 mJ or more, with respect to the surface of rat liver. On the other hand, in terms of ease in handling, such adhesion energy is preferably 10 mJ or less. Moreover, with regard to degradability, the weight reduction percentage (a sample weight after 1 month/a sample weight when is embedded) is preferably 60% or more, and more preferably 75% or more, within 1 month (28 days). It is to be noted that such adhesion energy can be measured according to the method described in Pharm Pharmaceut Sci (www.ualberta.ca/~csps) 3(3): 303-311, 2000.

### <Production method>

Next, a method for producing the chitosan/collagen structure of the present invention will be described. The method for producing the structure of the present invention is not particularly limited, as long as it applies the method of the present invention. The chitosan/collagen structure of the present invention can be produced in accordance with the examples described in JP Patent Publication (Kokoku) No. 63-59706 B (1988) and the method described in JP Patent Publication (Kokoku) No. 56-133344 B (1981), for example.

For instance, chitosan having a deacetylation degree of 50% to 70% and a collagen are dissolved in a solvent in such an amount that the weight ratio of chitosan and collagen is 30/70 or more and less than 70/30, so as to produce a solution. Subsequently, the obtained solution is dried to produce the structure of the present invention.

When the structure of the present invention is used for medical purpose, a residual organic solvent is problematic. The amount of such organic solvent remaining in the structure is preferably 1% or less, more preferably 0.1% or less, and most preferably 0.01% or less. Deacidification treatment methods that may be used herein include a method of treating the structure with an alkaline solution of sodium hydroxide, potassium hydroxide and the like, and a method of treating the structure with gas containing another solvent (such as water or alcohol) having affinity for the organic solvent.

### <Stmctufe>

The form of the structure is not particularly limited. Examples of the form of the structure include a gel, a sponge, a film, a non-woven fabric, a fiber (a tube), and a particle. A film or a sponge is preferable. Such sponge is generally composed of a homogeneous porous structure having a diameter of 10 to 200 µm as a mean pore size and a density of 0.05 or less.

As the shape of the structure, any shape may be applied. For example, pyramidal, conical, prismatic, cylindrical, spherical, and fusiform structures, and structures produced from any given forms are used. Prismatic, cylindrical, and fusiform structures, and structures produced from any given forms are preferable. Pyramidal, conical, prismatic, and cylindrical structures are more preferable. Prismatic and cylindrical structures are most preferable. The size of the structure is not particularly limited. In the case of a gel, a sponge, and a nonwoven fabric, 500 cm square or less is preferable. The size of the structure is more preferably 100 cm or less, particularly preferably 50 cm or less, and most preferably 10 cm or less. In the case of a fiber (a tube), the diameter (or a side) of such fiber or tube is 1 nm or more and 10 cm or less, preferably 1 nm or more and 1 cm or less, more preferably 1 nm or more and 100 µm or less, particularly preferably 1 nm or more and 1 µm or less, and most preferably 1 nm or more and 10 nm or less. In addition, the length is not particularly limited. It is preferably 10 µm or more and 100 m or less, more preferably 100 µm or more and 10 m or less, further preferably 1 mm or more and 1 m or less, and most preferably 1 cm or more and 30 cm or less. When the structure is a particle, it has a diameter of preferably 1 nm to 1 mm, more preferably 10 nm to 200 µm, further preferably 50 nm to 100 µm and particularly preferably 100 nm to 10 µm.

The thickness of the structure is not particularly limited. It is preferably 1 nm or more, more preferably 10 nm or more, further preferably 100 nm or more, further more preferably 1 µm or more, still further preferably 10 µm or more, and most preferably 100 µm or more.

Additives may be added to the composition, as necessary. Examples of such additives include a medicine, a coloring agent, a softener, a percutaneous absorption promoting agent, a moisturizing agent, a surfactant, an antiseptic, an aroma chemical, and a pH adjuster.

Specific examples of a medicine includes anticancer agents (for example, paclitaxel, topotecin, taxotere, and 5-fluorouracil), immunosuppressive agents (for example, rapamycin, tacrolimus, and cyclosporine), anti-inflammatory agents, antithrombotic agents, antipsychotic agents, antidepressive agents, antioxidants, antiallergic agents, growth factors, hormone, supplement ingredients, and cosmetic ingredients.

The intended use of the composition is not particularly limited. It is used as an adhesion preventive material, a wound-coating material, a transdermal agent, a local treatment agent, an oral treatment agent, a cosmetic product, a supplement, a food product, and a coloring material. It is preferably used as an adhesion preventive material, a wound-coating material, a transdermal agent, a local treatment agent, an oral treatment agent, and a cosmetic product. It is more preferably used as an adhesion preventive material, a transdermal agent, a local treatment agent an oral treatment agent, and a wound-coating material. It is most preferably used as an adhesion preventive material, a transdermal agent, a local treatment agent, and a wound-coating material.

The present invention will be specifically described in the following examples. However, these examples are not intended to limit the scope of the present invention.

### Example

### (I) Preparation of materials

### (1) Preparation of chitosan powder having a deacetylation degree of 60%

The chitin powder which was purified from crab shell was treated with 40% sodium hydroxide aqueous solution so as to obtain chitosan powder having a deacetylation degree of 60%.

### (2) Preparation of chitosan powder having a deacetylation degree of 90%

The chitin powder which was purified from crab shell was treated with 40% sodium hydroxide aqueous solution so as to obtain chitosan powder having a deacetylation degree of 90%.

### (3) Preparation of recombinant gelatin

The following recombinant gelatin CBE3 was prepared in accordance with the methods described in EP1014176A and WO04/08547

**Table 1:**

| Recombinant Gelatin | SEQ ID. NO. | RGD (/1molecule) | Percentage of polar amino acid (%) | Percentage of non-charged polar amino acid in polar amino acid (%) | Percentage of serine, glutamine, and asparagine (%) |
|---|---|---|---|---|---|
| CBE3 | 1 | 1 2 | 23.5 | 8.9 | 2.1 |

| | | | | | |
|---|---|---|---|---|---|
| CBE3 (SEQ ID NO.1): Molecular weight : 51.6kD Structure: [(GXY)64]3 Number of amino acids : 576 (GAPGAPGLQGAPGLQGMPGERGAAGLPGPKGERGDAGPKGADGAPGAPGLQGMP GERGAAGLPGPKGERGDAGPKGADGAPGKDGVRGLAGPIGPPGERGAAGLPGPKGE RGDAGPKGADGAPGKDGVRGLAGPIGPPGPAGAPGAPGLQGMPGERGAAGLPGPKG ERGDAGPKGADGAPGKDGVRGLAGPP)3 | | | | | |

### (II) Preparation of sponge

### (Comparative Example 1)

2g of chitosan powder having a deacetylation degree of 60% was gradually added in 100g of an aqueous solution of 2% by weight of acetic acid, and the mixture was stirred at 40°C for 2 hours for dissolution. The solution was left stand for defoaming. The resultant solution was poured into a plastic tray (10cm x10 cm, 1cm in depth), and was freeze-dxied at -40°C for 6 hours, and then was vacuum-dried for 24 hours, so as to obtain a sponge sheet.

### (Comparative Example 2)

0.46g of chitosan powder having a deacetylation degree of 60% and 1.54 g of gelatin (CBE3) were gradually added in 100g of an aqueous solution of 2% by weight of acetic acid, and the mixture was stirred at 40°C for 2 hours for dissolution. The solution was left stand for defoaming. The resultant solution was freeze-dried in the same way as in Comparative Example 1 so as to obtain a sponge sheet.

### (Example 1)

0.60g of chitosan having a deacetylation degree of 60% and 1.40 g of gelatin (CBE3) were gradually added in 100g of an aqueous solution of 2% by weight of acetic acid, and the mixture was stirred at 40°C for 2 hours for dissolution. The solution was left stand for defaming. The resultant solution was freeze-dried in the same way as in Comparative Example 1 so as to obtain a sponge sheet.

### (Example 2)

1.00g of chitosan having a deacetylation degree of 60% and 1.00 g of gelatin (CBE3) were gradually added in 100g of an aqueous solution of 2% by weight of acetic acid, and the mixture was stirred at 40°C for 2 hours for dissolution. The solution was left stand for defoaming. The resultant solution was freeze-dried in the same way as in Comparative Example 1 so as to obtain a sponge sheet

### (Example 3)

1.20g of chitosan having a deacetylation degree of 60% and 0.80 g of gelatin (CBE3) were gradually added in 100g of an aqueous solution of 2% by weight of acetic acid, and the mixture was stirred at 40ºC for 2 hours for dissolution. The solution was left stand for defoaming. The resultant solution was freeze-dried in the same way as in Comparative Example 1 so as to obtain a sponge sheet.

### (Comparative Example 3)

1.50g of chitosan having a deacetylation degree of 60% and 0.50 g of gelatin (CBE3) were gradually added in 100g of an aqueous solution of 2% by weight of acetic acid, and the mixture was stirred at 40°C for 2 hours for dissolution. The solution was left stand for defoaming. The resultant solution was freeze-dried in the same way as in Comparative Example 1 so as to obtain a sponge sheet.

### (Comparative Example 4)

1.00g of chitosan powder having a deacetylation degree of 90% and 1.00 g of gelatin (CBE3) were gradually added in 100g of an aqueous solution of 2% by weight of acetic acid, and the mixture was stirred at 40ºC for 2 hours for dissolution. The solution was left stand for defoaming. The resultant solution was freeze-dried in the same way as in Comparative Example 1 so as to obtain a sponge sheet.

### (III) Evaluation of sponge

Using the sponge sheets prepared in Examples 1 to 4 and Comparative Examples 1 to 3, the tissue adhesion property and the degradability were evaluated by the following methods. The results of evaluation are shown in Table 2. As is understood from the results of Table 2, good results were obtained in both the tissue adhesion property and the degradability in Examples 1 to 4 of the present invention.

### Evaluation of tissue adhesion property:

Liver was resected from Sprague-Dawley rat (SD rat) (male, 10 weeks old). For prevention of drying, a physiological saline was sprayed on a surface of the liver. A sample having a diameter of 10mm was placed on a surface of the liver, and 50g weight was applied for 1 minutes to adhere the sample. Then, the adhesive strength with the sample was measured using a tensile testing machine.

### Evaluation of degradability:

A sample having a diameter of 10mm and a thickness of 2mm was implanted into dorsal subcutaneous tissue of SD rat (male, 10 weeks old), and was taken out 28 days later. The weight decrease ratio [(sample weight after 1 month)/(weight at implantation)] was measured.

**Table 2:**

| chitosan (deacetylation degree: 60%) | chitosan (deacetylation degree: 90%) | gelatin | Tissue adhesion property [adhesion energy: mJ] | Degradability at 28^{th} day | Remarks |
|---|---|---|---|---|---|
| 23 | | 77 | 1.21mJ(×) | 90%(○) | Comparative Example 2 |
| 30 | | 70 | 2.08mJ(○) | 85%(○) | Example 1 |
| 50 | | 50 | 2.20mJ(○) | 83%(○) | Example 2 |
| 60 | | 40 | 2.23mJ(○) | 78%(○) | Example 3 |
| 75 | | 25 | 0.32mJ(×) | 55%(×) | Comparative Example 3 |
| 100 | | 0 | 2.72mJ(○) | 42%(×) | Comparative Example 1 |
| | 50 | 50 | 2.19mJ(○) | 54%(×) | Comparative Example 4 |

| | | | | | |
|---|---|---|---|---|---|
| Note 1: the vales for chitosan and gelatin indicate a ratio by weight. Note 2: ○ means a good result, and × means a bad result. | | | | | |

The same experiment was carried out using acid-treated gelatin (PSP gelatin, NIPPI Inc.) in place of the recombinant gelatin of the aforementioned Examples. As a result, the similar results were obtained.

## Claims

1. A structure comprised of a mixture of chitosan and collagen, wherein (i) the deacetylation degree of chitosan is 50% to 70%, (ii) the weight ratio of chitosan and collagen is 30/70 or more and less than 70/30.

2. The structure of claim 1 wherein the collagen is a recombinant collagen.

3. The structure of claim 1 wherein the weight ratio of chitosan and collagen is 33/67 or more and 60/40 or less.

4. The structure of claim 1, which is produced by dissolving chitosan having a deacetylation degree of 50% to 70% and a collagen in a solvent in such an amount that the weight ratio of chitosan and collagen is 30/70 or more and less than 70/30, so as to produce a solution, and drying the solution.

5. A method for producing the structure of claim 1, which comprises dissolving chitosan having a deacetylation degree of 50% to 70% and a collagen in a solvent in such an amount that the weight ratio of chitosan and collagen is 30/70 or more and less than 70/30, so as to produce a solution, and drying the solution.
